# EUROPEAN PATENT APPLICATION

(11) **EP 4 318 480 A1**
(43) Date of publication of application: **07.02.2024**
(21) Application number: 22791779.6
(22) Date of filing: 21.04.2022
(51) Int. Cl.: G16C 20/30, G06N 20/00

(54) **CHARACTERISTICS PREDICTION SYSTEM, CHARACTERISTICS PREDICTION METHOD, AND CHARACTERISTIC PREDICTION PROGRAM**

(30) Priority: 23.04.2021 JP 2021073162
(71) Applicant: RESONAC CORPORATION, Tokyo 105-7325 (JP)
(72) Inventor: HANAOKA Kyohei, Tokyo 105-7325 (JP)
(74) Representative: Berggren Oy
(86) International application number: PCT/JP2022/018415
(87) International publication number: WO 2022/225008

(57) **Abstract**

An input data generation system is a system generating input data for machine learning for predicting the properties of a material based on a plurality of raw materials having a known partial structure, and includes a processor. The processor receives the input of partial structure data for specifying the known partial structure of each of the plurality of raw materials and blending ratio data indicating a ratio of the blending of each of the plurality of raw materials, generates partial structure input data indicating the known partial structure, on the basis of the partial structure data for each of the plurality of raw materials, generates synthetic input data by reflecting the blending ratio data relevant to the plurality of raw materials on the partial structure input data and compiling the partial structure input data, and inputs the synthetic input data to a machine learning model.

## Description

### Technical Field

One aspect of the present disclosure relates to a property prediction system, a property prediction method, and a property prediction program.

### Background Art

In the related art, the structure of a molecule is acquired in a predetermined format, converted into vector information, and input to a machine learning algorithm to predict properties. For example, a method for predicting connectivity between a three-dimensional structure of a biological polymer and a three-dimensional structure of a compound by using machine learning is known (refer to Patent Literature 1 described below). In this method, a predicted three-dimensional structure of a composite body of the biological polymer and the compound is generated on the basis of the three-dimensional structure of the biological polymer and the three-dimensional structure of the compound, the predicted three-dimensional structure is converted into a predicted three-dimensional structure vector, and the predicted three-dimensional structure vector is determined by using a machine learning algorithm to predict the connectivity between the three-dimensional structure of the biological polymer and the three-dimensional structure of the compound.

### Citation List

### Patent Literature

Patent Literature 1: Japanese Unexamined Patent Publication No. 2019-28879

### Summary of Invention

### Technical Problem

Recently, a technology of predicting the properties of a material by a neural network to which data indicating a structure, such as a molecular graph relevant to a material with a clear structure, is input has been known. However, in a case where only a part of a chemical structure to be the input of the machine learning is clear, the prediction of the properties of the material is not attained by learning a plurality of incomplete partial structures. Therefore, there is a need for a mechanism for efficiently predicting the properties of a material in which only a part of the chemical structure is clear.

### Solution to Problem

A property prediction system according to one aspect of the present disclosure is a property prediction system predicting the properties of a material based on a plurality of raw materials having a known partial structure, and includes at least one processor, in which at least one processor is configured to receive at least the input of partial structure data for specifying the known partial structure of each of the plurality of raw materials and blending ratio data indicating a ratio of the blending of each of the plurality of raw materials, generate partial structure input data indicating the known partial structure, on the basis of the partial structure data for each of the plurality of raw materials, reflect blending ratio data relevant to the plurality of raw materials on partial structure input data of the plurality of raw materials, and input input data based on partial structure input data for each of the plurality of raw materials on which blending ratio data is reflected to a machine learning model.

Alternatively, a property prediction method of another aspect of the present disclosure is a property prediction method for predicting the properties of a material based on a plurality of raw materials having a known partial structure, which is executed by a computer including at least one processor, and includes a step of receiving at least the input of partial structure data for specifying the known partial structure of each of the plurality of raw materials and blending ratio data indicating a ratio of the blending of each of the plurality of raw materials, a step of generating partial structure input data indicating the known partial structure, on the basis of the partial structure data for each of the plurality of raw materials, a step of reflecting blending ratio data relevant to the plurality of raw materials on partial structure input data of the plurality of raw materials, and a step of inputting input data based on partial structure input data for each of the plurality of raw materials on which blending ratio data is reflected to a machine learning model.

Alternatively, a property prediction program of another aspect of the present disclosure is a property prediction program for predicting the properties of a material based on a plurality of raw materials having a known partial structure, and allows a computer to execute a step of receiving at least the input of partial structure data for specifying the known partial structure of each of the plurality of raw materials and blending ratio data indicating a ratio of blending of each of the plurality of raw materials, a step of generating partial structure input data indicating the known partial structure, on the basis of the partial structure data for each of the plurality of raw materials, a step of reflecting blending ratio data relevant to the plurality of raw materials on partial structure input data of the plurality of raw materials, and a step of inputting input data based on the partial structure input data for each of the plurality of raw materials on which blending ratio data is reflected to a machine learning model.

According to the aspects described above, partial structure input data expressing a known partial structure is generated on the basis of the partial structure data for each of the plurality of raw materials, the ratio of the blending relevant to the plurality of raw materials is reflected on partial structure input data of the plurality of raw materials, and the input data based on partial structure input data for each of the plurality of raw materials on which the ratio is reflected is input to the machine learning model. As a result thereof, by processing the input data with the machine learning for the material to be produced on the basis of the plurality of raw materials in which only a part of the chemical structure is clear, it is possible to efficiently predict the properties of the material.

### Advantageous Effects of Invention

According to the aspect of the present disclosure, it is possible to efficiently predict the properties of the material to be produced on the basis of the raw material in which only a part of the chemical structure is clear.

### Brief Description of Drawings

FIG. 1 is a diagram illustrating an example of a hardware configuration of a computer configuring a property prediction system according to an embodiment.
FIG. 2 is a diagram illustrating an example of a functional configuration of the property prediction system according to the embodiment.
FIGS. 3A and 3B are diagrams illustrating an example of a partial structure specified by partial structure data acquired by an acquisition unit 11 in FIG. 2.
FIG. 4 is a flowchart illustrating an example of an operation of the property prediction system according to the embodiment.
FIG. 5 is a diagram illustrating an example of a functional configuration of a property prediction system according to a Modification Example.
FIGS. 6A and 6B are diagrams illustrating a specific example of partial structure input data generated by a generation unit 12 in FIG. 5.

### Description of Embodiments

Hereinafter, an embodiment of the present invention will be described in detail, with reference to the attached drawings. Note that, in the description, the same reference numerals will be used in the same constituents or constituents with the same function, and the repeated description will be omitted.

### [Outline of System]

A property prediction system 1 according to an embodiment is a computer system executing prediction processing of the properties of a multi-component substance, which is a material produced by blending a plurality of raw materials having a known partial structure at various ratios, by using a machine learning model. The raw material indicates a chemical substance in which at least a part of the molecular structure is clear and a chemical substance in which the structure is completely unclear, which are used to generate the multi-component substance, and for example, is a monomer, a polymer, a low-molecular additive, and a monomolecule such as a solute molecule and a gas molecule. A plurality of types of molecules may be contained in one raw material. The multi-component substance is a chemical substance generated by blending a plurality of raw materials at a predetermined ratio, and for example, in a case where the raw material is a monomer or a polymer, the multi-component substance is a polymer alloy, in a case where the raw material is a solute molecule or a solvent, the multi-component substance is a mixed solution, and in a case where the raw material is a gas molecule, the multi-component substance is a mixed gas.

A prediction processing target of the property prediction system 1 is the properties of the multi-component substance. For example, in a case where the multi-component substance is a resin, the properties of the multi-component substance are a glass transition temperature, thermophysical properties such as a melting point, mechanical properties, bonding adhesiveness, and the like. In addition, in a case where the multi-component substance is another type of substance, the properties of the multi-component substance are the medicinal effect or the toxicity of a medical agent, riskiness such as the ignition point of a combustible material, appearance properties, suitability for a specific use, and the like. Machine learning is used in the prediction processing of the property prediction system 1. The machine learning is a method for autonomously finding out a law or a rule, on the basis of given information. A specific method of the machine learning is not limited. For example, the machine learning may be machine learning using a machine learning model, which is a computation model. More specifically, the computation model is a neural network. The neural network indicates an information processing model imitating the mechanism of the human cerebral nerve system. More specific examples of other computation models may include support vector regression (SVR), random forest, and the like, in addition to the neural network.

### [Configuration of System]

The property prediction system 1 includes one or more computers. In the case of using a plurality of computers, such computers are connected via a communication network such as the internet or the intranet to logically construct one property prediction system 1.

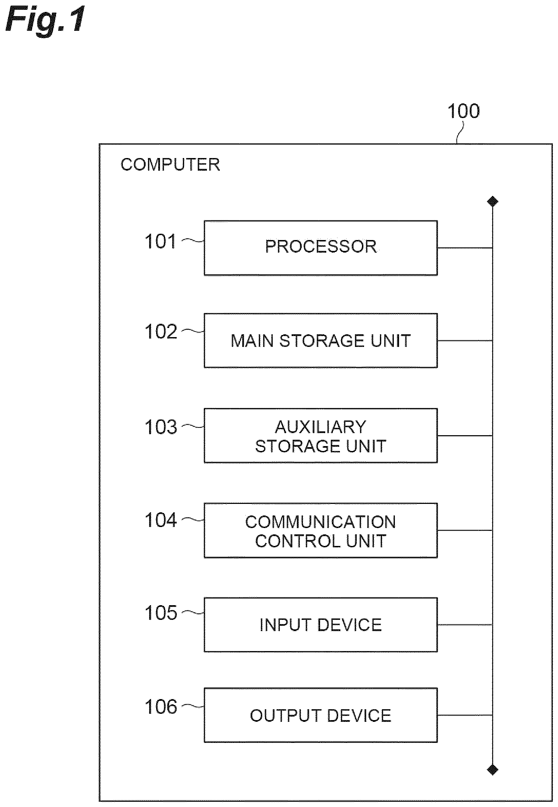

FIG. 1 is a diagram illustrating an example of the general hardware configuration of a computer 100 configuring the property prediction system 1. For example, the computer 100 includes a processor (for example, a CPU) 101 executing an operating system, an application program, or the like, a main storage unit 102 including a ROM and a RAM, an auxiliary storage unit 103 including a hard disk, a flash memory, and the like, a communication control unit 104 including a network card or a wireless communication module, an input device 105 such as a keyboard, a mouse, and a touch panel, and an output device 106 such as a monitor and a touch panel display.

Each functional constituent of the property prediction system 1 is attained by reading a program set in advance on the processor 101 or the main storage unit 102 and allowing the processor 101 to execute the program. The processor 101 operates the communication control unit 104, the input device 105, or the output device 106 and reads and writes data in the main storage unit 102 or the auxiliary storage unit 103, in accordance with the program. Data or a database required for processing is stored in the main storage unit 102 or the auxiliary storage unit 103.

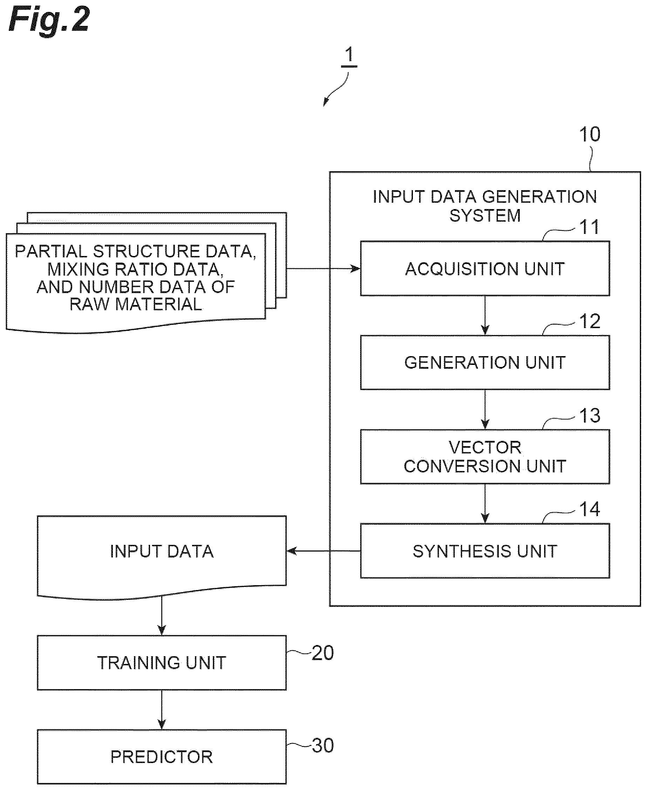

FIG. 2 is a diagram illustrating an example of the functional configuration of the property prediction system 1. The property prediction system 1 includes an input data generation system 10, a training unit 20, and a predictor 30. The input data generation system 10, the training unit 20, and the predictor 30 may be constructed on the same computer 100, or a part thereof may be constructed on another computer 100. First, the functional configuration of the input data generation system 10 will be described. The input data generation system 10 includes an acquisition unit 11, a generation unit 12, a vector conversion unit 13, and a synthesis unit 14, as a functional constituent.

The acquisition unit 11 is a functional constituent receiving the input of partial structure data relevant to the known partial structure of each molecule configuring the plurality of raw materials to be the basis of the multi-component substance, which is a prediction target, blending ratio data indicating a ratio of the blending of each of the raw materials when it is assumed that the plurality of raw materials are blended to produce the multi-component substance, and number data indicating the number of known partial structures in the molecules of the plurality of raw materials. The acquisition unit 11 may acquire such data from the database in the input data generation system 10, in accordance with the selection input of the user of the input data generation system 10, or may acquire the data, in accordance with the selection of the user from the external computer or the like. In addition, the acquisition unit 11 may acquire such data by the direct input of the user.

Specifically, the acquisition unit 11 acquires at least first partial structure data for specifying a partial structure included in the molecules of a first raw material, and second partial structure data for specifying a partial structure included in the molecules of a second raw material. Such partial structure data is molecular structure information indicating a partial structure. For example, such partial structure data may be data for specifying the molecular structure with a number, an alphabetic character, a text, a vector, and the like, may be data visualizing the molecular structure with two-dimensional coordinates, three-dimensional coordinates, and the like, or may be data of any combination of two or more data pieces described above. Each numerical value configuring the partial structure data may be represented by a decimal system, or may be represented by other notation methods such as a binary system and a hexadecimal system. More specifically, such partial structure data may be a structural formula, a molecular graph, data in a simplified molecular input line entry system (SMILES) notation, data in a MOL file format, and the like.

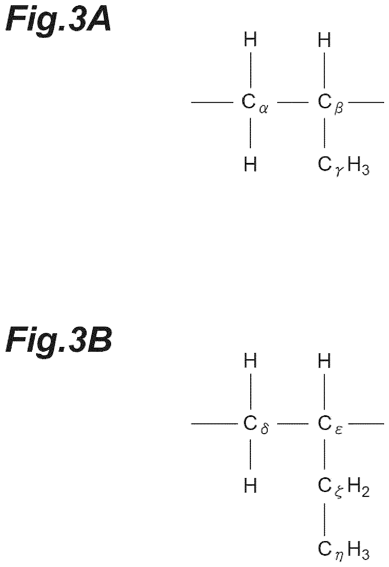

FIGS. 3A and 3B illustrate an example of a partial structure specified by the first partial structure data, in which FIG. 3A illustrates an example of the partial structure, and FIG. 3B illustrates another example of the partial structure. As described above, the partial structure data of each of the raw materials may include data for specifying a plurality of partial structures. The first partial structure data is data capable of specifying each of one or more partial structures in the molecules of the first raw material. Similarly, the second partial structure data is data capable of specifying one or more partial structures in the molecules of the second raw material.

In addition, as the blending ratio data indicating a ratio r of the plurality of raw materials, the acquisition unit 11 may acquire data indicating a ratio itself of each of the raw materials, may acquire data indicating a ratio between the plurality of raw materials, or may acquire data indicating the blending amount (the weight, the volume, and the like) of each of the plurality of raw materials in an absolute value or a relative value. For example, the acquisition unit 11 acquires Ratio r₁ = "0.5" of a first monomer, which is the first raw material, and Ratio r₂ = "0.5" of a second monomer, which is the second raw material.

Further, the acquisition unit 11, for example, acquires data indicating the number "1" of partial structures, illustrated in FIG. 3A, in the molecules of the first raw material, and the number "1" of partial structures, illustrated in FIG. 3B, in the first raw material, as the number data relevant to the number of known partial structures in the molecules of the plurality of raw materials. Similarly, the acquisition unit 11 acquires data indicating the number of partial structures in the molecules of each of the plurality of raw materials. Note that, the number data acquired here may be data indicating a number normalized such that the total number of partial structures in the molecules of the same raw material is "1".

The generation unit 12 generates partial structure input data D0 in which the partial structure data relevant to the partial structure included in the raw material, the data of the ratio r relevant to the raw material, and the number data relevant to the partial structure are combined, for each of the partial structures of the plurality of raw materials, on the basis of the partial structure data, the blending ratio data, and the number data acquired by the acquisition unit 11. Then, the generation unit 12 repeats the generation of the partial structure input data D0 for all the partial structure data pieces for each of the plurality of raw materials.

The vector conversion unit 13 converts each of all the partial structure input data pieces D0 generated by the generation unit 12 into one vector data piece. For example, the vector conversion unit 13 performs the conversion to a vector V_{M} by molecular description, with reference to the molecular structure relevant to each of the partial structure data pieces included in the partial structure input data D0. According to the molecular description, molecular characteristics indicated by the partial structure data can be represented as a numerical string, on the basis of the chemical structure thereof. Any method can be adopted as a molecular description method insofar as the molecular structure is vectorized by the method, and for example, Extended Connectivity FingerPrints (ECFP), MACCS FingerPrints, PubChem FingerPrints, Substructure FingerPrints, Estate FingerPrints, BCI FingerPrints, Molprint2D FingerPrints, Pass base FingerPrints, and the like can be adopted. Further, the vector conversion unit 13 generates partial structure input data D by combining the data of the ratio r relevant to the raw material having a partial structure, and the number data of the partial structure in the raw material, with the vector V_{M} generated for each of the partial structure data pieces.

The synthesis unit 14 compiles the vector V_{M} for each of all the partial structures for each of the plurality of raw materials, which is converted by the vector conversion unit 13, on one vector data piece to generate synthetic input data F. For example, in a case where there are two partial structure input data pieces D_{1,1} and D_{1,2} corresponding to the first raw material, and two partial structure input data pieces D_{2,1} and D_{2,2} corresponding to the second raw material, the synthesis unit 14 generates the synthetic input data F in which four vectors V_{M} corresponding to such four partial structure input data pieces D_{1,1}, D_{1,2}, D_{2,1}, and D_{2,2} are compiled.

In this case, the synthesis unit 14 reflects the blending ratio data and the number data corresponding to each of the partial structures on four vectors V_{M} corresponding to such four partial structure input data pieces D_{1,1}, D_{1,2}, D_{2,1}, and D_{2,2} to obtain the weighted average of four vectors V_{M} and generate the synthetic input data F. More specifically, the synthesis unit 14 multiplies the element of each of four vectors V_{M} corresponding to the partial structure input data pieces D_{1,1}, D_{1,2}, D_{2,1}, and D_{2,2} by the ratio r and the number n corresponding to each of the partial structure, and then, adds (or averages) the element of each of four vectors V_{M} to generate the synthetic input data F. For example, the synthesis unit 14 multiplies the vector V_{M} corresponding to the partial structure of the first raw material by a value obtained by multiplying a ratio r₁ of the first raw material and the number n of the partial structure together, and multiplies the vector V_{M} corresponding to the partial structure of the second raw material by a value obtained by multiplying a ratio r₂ of the second raw material and the number n of the partial structure together. Here, the reflection of the ratio and the number data may be performed by adding the value obtained by multiplying the ratio r and the number n together to each of the elements of the vector V_{M}, or may be performed by concatenating the value obtained by multiplying the ratio r and the number n together to each of the elements. In addition, more generally, the synthesis unit 14 may generate a vector by using a function of outputting a single vector in accordance with a certain rule, with the vector, the blending ratio, and the number relevant to all the partial structures as input, or may generate a single vector in one processing without dividing the processing into a step of reflecting the blending ratio and a step of adding the vector.

Further, the synthesis unit 14 outputs the generated synthetic input data F to the outside such that the data is input to an external machine learning model. That is, the output synthetic input data F is read by the training unit 20 in a computer connected to the outside of the input data generation system 10. Then, in the training unit 20, a learned model is generated by inputting the synthetic input data F to the machine learning model as an explanatory variable, together with any training label. Further, a machine learning model in the predictor 30 is set on the basis of the learned model generated by the training unit 20. Here, the training unit 20 and the predictor 30 may be the same functional unit. Then, by inputting the synthetic input data F generated by the input data generation system 10 to the machine learning model in the predictor 30, the predictor 30 generates and outputs a prediction result of the properties of the multi-component substance. Note that, the training unit 20 and the predictor 30 may be configured in the same computer as the computer 100 configuring the input data generation system 10, or may be configured in a computer different from the computer 100.

In an example, the training unit 20 generates a learned model using a neural network. The learned model is generated by a computer processing training data including a plurality of combinations of input data and output data. The computer calculates the output data by inputting the input data to the machine learning model, and obtains an error between the calculated output data and the output data indicated by the training data (that is, an error between an estimation result and a ground truth). Then, the computer updates given parameters of the neural network, which is the machine learning model, on the basis of the error. The computer generates the learned model by repeating such learning. The processing of generating the learned model can be referred to as a learning phase, and the processing of the predictor 30 using the learned model can be referred to as an operation phase.

### [Operation of System]

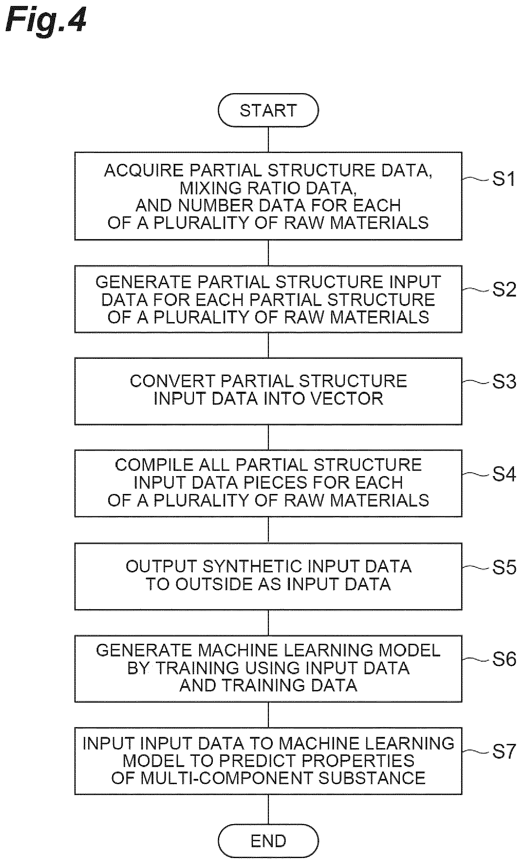

The operation of the property prediction system 1 will be described, and a property prediction method according to this embodiment will be described, with reference to FIG. 4. FIG. 4 is a flowchart illustrating an example of the operation of the property prediction system 1.

First, in a case where input data generation processing is started in accordance with the instruction input of the user of the input data generation system 10, the acquisition unit 11 is allowed to acquire the partial structure data, the blending ratio data, and the number data for each of the plurality of raw materials (step S1). Next, the generation unit 12 is allowed to generate the partial structure input data D0 for each of the partial structures of the plurality of raw materials, on the basis of each of the partial structure data pieces (step S2). After that, the vector conversion unit 13 is allowed to convert each of all the partial structure input data pieces D0 into one vector V_{M} in a vector format, and combine the data of the ratio r relevant to the raw material having the partial structure, and the number data of the partial structure in the raw material, with the vector V_{M}, to generate the partial structure input data D (step S3).

Next, the synthesis unit 14 is allowed to compile the vectors V_{M} corresponding to all the partial structure input data pieces D for each of the plurality of raw materials to generate the synthetic input data F (step S4). In this case, the synthesis unit 14 is allowed to compute the weighted average of the vectors V_{M} while reflecting the blending ratio data and the number data on each of the vectors V_{M} to generate the synthetic input data F. After that, the synthesis unit 14 is allowed to output the synthetic input data F to the training unit 20 as input data for machine learning (step S5). In this case, the reflection of the ratio and the number on the vector V_{M} is performed by multiplying, adding, or concatenating the value obtained by multiplying the ratio and the number together with respect to each of the vectors V_{M}. In addition, more generally, the synthesis unit 14 may generate the vector by using the function of outputting the single vector in accordance with a certain rule, with the vector, the blending ratio, and the number relevant to all the partial structures as input, or may generate the single vector in one processing without dividing the processing into the step of reflecting the blending ratio and the step of adding the vector.

Next, in the training unit 20, the learning phase is executed, and the learned model is generated by learning using the input data and the training data (step S6). Then, the generated learned model is set in the predictor 30, and the predictor 30 is allowed to execute the operation phase by using the input data newly acquired from the input data generation system 10, and generate and output the prediction result of the properties of the multi-component substance (step S7).

### [Program]

A property prediction program for allowing a computer or a computer system to function as the property prediction system 1 includes a program code for allowing the computer system to function as the acquisition unit 11, the generation unit 12, the vector conversion unit 13, the synthesis unit 14, the training unit 20, and the predictor 30. Such a property prediction program may be provided by being regularly recorded in a physical recording medium such as a CD-ROM, a DVD-ROM, and a semiconductor memory. Alternatively, the property prediction program may be provided via a communication network as a data signal superimposed on a carrier wave. The provided property prediction program, for example, is stored in the auxiliary storage unit 103. By the processor 101 reading and executing the property prediction program from the auxiliary storage unit 103, each of the functional constituents described above is attained.

### [Effect]

As described above, according to the embodiment described above, the partial structure input data D expressing the known partial structure is generated on the basis of the partial structure data for each of the plurality of raw materials, the ratio relevant to the plurality of raw materials is reflected on the partial structure input data D of the plurality of raw materials, and the partial structure input data D for each of the plurality of raw materials is compiled on the synthetic input data F and input to the machine learning model. As a result thereof, by learning the partial structure information with the machine learning for the multi-component substance to be produced on the basis of the plurality of raw materials in which only a part of the chemical structure is clear, it is possible to efficiently predict the properties of the substance.

In addition, in the embodiment described above, the partial structure data for specifying the known partial structure in the molecules configuring each of the plurality of raw materials, and the number of known partial structures in the molecules are received, and the input data to be input to the machine learning model is generated by reflecting a value obtained by multiplying the blending ratio data for each of the plurality of raw materials and the number of known partial structures together on the partial structure input data D for each of the plurality of raw materials. In this case, when the partial structure in the molecules of the raw material and the number thereof are clear, it is possible to reflect the ratio of the raw material and the number of the partial structure on the partial structure input data D. As a result thereof, it is possible to accurately predict the properties of the multi-component substance based on the raw material.

In addition, in the embodiment described above, the partial structure input data D0 is generated as the molecular structure data of the known partial structure. As a result thereof, it is possible to efficiently generate the partial structure input data D0.

Further, in the embodiment described above, the input data to be input to the machine learning model is generated by multiplying, adding, or concatenating a value based on the blending ratio data for each of the plurality of raw materials with respect to a plurality of vectors included in the partial structure input data D for each of the plurality of raw materials, and compiling the plurality of multiplied, added, or concatenated vectors on one vector. Accordingly, it is possible to simply and effectively reflect the ratio of the raw material on the partial structure input data D for each of the raw materials. As a result thereof, the prediction accuracy of the properties of the multi-component substance is improved.

### [Modification Example]

The present invention has been described in detail, on the basis of the embodiment. However, the present invention is not limited to the embodiment described above. The present invention can be variously modified within a range not departing from the gist thereof.

In the embodiment described above, an example has been described in which the input data generation system 10 generates the synthetic input data F by combining the partial structure input data D of two raw materials, but the partial structure input data D of three or more raw materials may be combined together with the ratio thereof.

In addition, a certain conversion rule provided in the vector conversion unit 13 of the input data generation system 10 may be other rules.

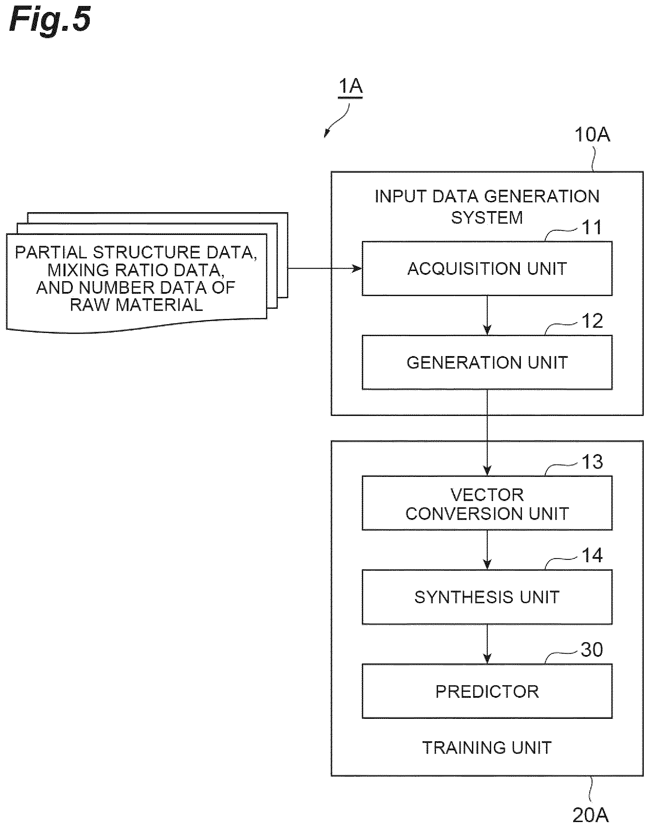

FIG. 5 illustrates the configuration of a property prediction system 1A according to a Modification Example. In this Modification Example, the functions of the vector conversion unit 13, the synthesis unit 14, and the predictor 30 are provided in a training unit 20A. Then, in the training unit 20A, a vectorization function, a data synthesizing function, and a reflection function using blending ratio data and number data of such functional units are attained by using a machine learning model of a neural network integrated with the predictor 30. In this case, the training unit 20A inputs the partial structure input data D0 generated by the generation unit 12 of an input data generation system 10A to the machine learning model.

Here, the training unit 20A inputs the partial structure input data D0 to the machine learning model using the neural network. The partial structure data included in the input partial structure input data D0 is a structural formula, a molecular graph, data in a SMILES notation, data in three-dimensional coordinates, and the like. Hereinafter, an example will be described in which a molecular graph is input to the machine learning model as the partial structure input data D0.

That is, in the Modification Example described above, the generation unit 12 generates a node vector set FV corresponding to a node set V in Molecular Graph G = (V,E) in a one-to-one manner, and an edge vector set FE corresponding to an edge set E in the molecular graph in a one-to-one manner, with reference to molecular graph data for each of the partial structures. The node vector is a vector for specifying atoms of a node, and for example, is a vector element in which numerical values (an atomic number, an electronegativity, and the like) indicating the characteristics of the atoms configuring the node that is each member of the set are sequentially arranged. The edge vector is a vector for specifying the properties of a bond between the nodes, and for example, is a vector element in which numerical values (a bond order, a bond distance, and the like) indicating the characteristics of an edge that is each member of the set are sequentially arranged. Further, the generation unit 12 generates the partial structure input data D0 by combining the original molecular graph data, the data of the ratio r relevant to the raw material having the partial structure described above, and the number data of the partial structure described above in the raw material with the node vector set FV and the edge vector set FE. Then, the generation unit 12 repeats the generation of the partial structure input data D0 for all the partial structure data pieces for each of the plurality of raw materials. In addition, the generation unit 12 may combine only the data of the ratio r and the number data with the molecular structure data, which is the molecular graph, to generate the partial structure input data D0.

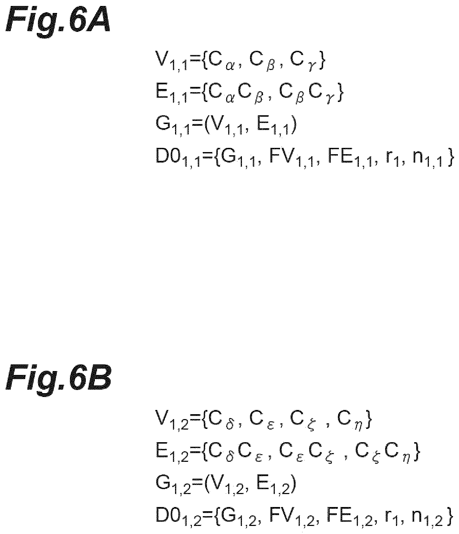

FIGS. 6A and 6B illustrate a specific example of the partial structure input data corresponding to the first raw material generated by the generation unit 12, in which FIG. 6A illustrates partial structure input data generated for the partial structure illustrated in FIG. 3A, and FIG. 6B illustrates partial structure input data generated for the partial structure illustrated in FIG. 3B. As described above, in a case where the partial structure illustrated in FIG. 3A is a target, the generation unit 12 generates Node Vector Set FV_{1,1} = {FC_{α}, FC_{β}, FC_{γ}} and Edge Vector Set FE_{1,1} = {FC_{α}C_{β}, FC_{β}C_{γ}}, and generates partial structure input data D0_{1,1} in which Molecular Graph G_{1,1} = (V_{1,1}, E_{1,1}), which is the original partial structure data, the data of the ratio r₁ relevant to the first raw material, the data of the number n_{1,1} of the molecular structure in the molecules of the first raw material are combined with such data. In the description of the vector sets FV and FE, each element in parentheses denoted by the initial letter F indicates a vector element after the vector conversion as described above (the same applies to the following). For example, the element FC_{α} of the node vector set FV_{1,1} is a vector element after being converted into a numerical value indicating the characteristics of a molecule C_{α} configuring a node. In addition, in a case where the partial structure illustrated in FIG. 3B is a target, the generation unit 12 generates Node Vector Set FV_{1,2} = {FC_{δ}, FC_{ε}, FC_{ζ}, FC_{η}} and Edge Vector Set FE_{1,2} = {FC_{δ}C_{ε}, FC_{ε}C_{ζ}, FC_{ζ}C_{η}}, and generates partial structure input data D0_{1,2} in which Molecular Graph G_{1,2} = (V_{1,2}, E_{1,2}), which is the original partial structure data, the data of the ratio r₁ relevant to the first raw material, and the data of the number n_{1,2} of the molecular structure in the molecules of the first raw material are combined with such data. Further, the generation unit 12 generates partial structure input data pieces D0_{2,1}, D0_{2,2}, ... for the partial structure corresponding to the second raw material.

The training unit 20A receives all the partial structure input data pieces D0 for each of the plurality of raw materials, and generates the synthetic input data F, which is one vector, on the basis of the partial structure input data D0, by the vector conversion unit 13 and the synthesis unit 14 attained by the machine learning model using the neural network. Then, the synthetic input data F is input to the predictor 30 in the same machine learning model. Note that, in this Modification Example, the function of the predictor 30 may be separated from the machine learning model of the training unit 20A and attained by another machine learning model.

In addition, the input data generation system 10 of the embodiment described above may have a function of merging and expressing the partial structure data, the blending ratio data, and the number data for each of the partial structures in one molecular graph. In this case, as with the generation unit 12 of the property prediction system 1A according to the Modification Example described above, the partial structure input data D0 is generated. Then, the blending ratio data and the number data are reflected on the molecular graph included in the partial structure input data D0 for each of the partial structures. Specifically, the blending ratio data and the number data are reflected on a feature vector included in the node vector set FV and the edge vector set FE included in the partial structure input data D0. Further, the synthetic input data F is generated by merging the molecular graph included in the partial structure input data D0 for each of the partial structures on which the blending ratio is reflected in one molecular graph data piece. In such a case, the input data generation system 10 attains the property prediction by inputting the generated molecular graph for each of the multi-component materials to the machine learning model capable of generating prediction data by the input of the molecular graph.

In addition, when converting the partial structure input data D for each of the plurality of raw materials into a one-dimensional vector, the vector conversion unit 13 of the input data generation system 10 of the embodiment described above may reflect a value indicating a difference in the raw materials on the vector. For example, the vector conversion unit 13 may concatenate a vector indicating a difference in the raw materials with a one-hot vector to the vector V_{M}. In addition, the vector conversion unit 13 may concatenate a vector indicating a difference in the raw materials with dispersion expression to the vector V_{M}. Accordingly, even in a case where the partial structures are the same, it is possible to reflect a difference in the raw materials on the partial structure input data D for each of the raw materials. As a result thereof, the prediction accuracy of the properties of the multi-component substance is further improved. In addition, the synthesis unit 14 may concatenate a vector in which the blending amounts of a raw material group in which all the structures are unknown are arranged to the synthetic input data F. Accordingly, even in the case of including a raw material in which the partial structure is also unknown, it is possible to predict the properties.

On the other hand, in the case of using a neural network with the graph as described in the Modification Example described above as input, and the vector indicating a difference in the raw materials is added, the input data generation system 10 of the embodiment described above may concatenate the vector to the node vector of each of the raw materials generated by the generation unit 12. Note that, the vector conversion unit 13 of the property prediction system 1A according to the Modification Example described above may be attained by the machine learning model of the neural network. In this case, the vector conversion unit 13 may be operated to reflect the vector indicating a difference in the raw materials on the vector of an intermediate layer of the neural network.

A processing procedure of an input data generation method executed by at least one processor is not limited to an example in the embodiment described above. For example, a part of the steps (the processing) described above may be omitted, or each of the steps may be executed in another order. In addition, any two or more steps among the steps described above may be combined, or a part of the steps may be corrected or deleted. Alternatively, other steps may be executed in addition to each of the steps described above. For example, the processing of steps S6 and S7 may be omitted.

In the present disclosure, an expression "at least one processor executes first processing, executes second processing, ..., and executes n-th processing.", or an expression corresponding thereto indicates a concept including a case where an executor (that is, the processor) of n processing pieces of the first processing to the n-th processing is changed in the middle. That is, the expression indicates a concept including both of a case where all of n processing pieces are executed by the same processor and a case where the processor is changed in n processing pieces, in accordance with any policy.

### Reference Signs List

1, 1A: property prediction system, 10, 10A: input data generation system, 100: computer, 101: processor, 11: acquisition unit, 12: generation unit, 13: vector conversion unit, 14: synthesis unit, 20: training unit, 30: predictor.

## Claims

1. A property prediction system predicting properties of a material based on a plurality of raw materials having a known partial structure, the system comprising
at least one processor,
wherein the at least one processor is configured to:
receive at least input of partial structure data for specifying the known partial structure of each of the plurality of raw materials and blending ratio data indicating a ratio of blending of each of the plurality of raw materials;
generate partial structure input data indicating the known partial structure, on the basis of the partial structure data for each of the plurality of raw materials;
reflect blending ratio data relevant to the plurality of raw materials on partial structure input data of the plurality of raw materials; and
input input data based on partial structure input data for each of the plurality of raw materials on which the blending ratio data is reflected to a machine learning model.

2. The property prediction system according to claim 1, wherein the at least one processor is configured to:
receive partial structure data for specifying the known partial structure in a molecule configuring each of the plurality of raw materials and the number of known partial structures in the molecule; and
reflect a value obtained by multiplying blending ratio data relevant to the plurality of raw materials and the number of known partial structures together on the partial structure input data of the plurality of raw materials.

3. The property prediction system according to claim 1 or 2,
wherein the partial structure input data is molecular structure information indicating a structure of the known partial structure.

4. The property prediction system according to any one of claims 1 to 3,
wherein the at least one processor
multiplies, adds, or concatenates a value based on blending ratio data for each of the plurality of raw materials with respect to a plurality of vectors based on the partial structure input data for each of the plurality of raw materials, and compiles the plurality of multiplied, added, or concatenated vectors on one vector to input the one vector to the machine learning model.

5. The property prediction system according to any one of claims 1 to 4,
wherein the at least one processor
further reflects a value indicating a difference in the raw materials on data, which is partial structure input data for each of the plurality of raw materials, and compiles the data on one data piece to input the one data piece to the machine learning model.

6. A property prediction method for predicting properties of a material based on a plurality of raw materials having a known partial structure, the method being executed by a computer including at least one processor, the method comprising:
a step of receiving at least input of partial structure data for specifying the known partial structure of each of the plurality of raw materials and blending ratio data indicating a ratio of blending of each of the plurality of raw materials;
a step of generating partial structure input data indicating the known partial structure, on the basis of the partial structure data for each of the plurality of raw materials;
a step of reflecting blending ratio data relevant to the plurality of raw materials on partial structure input data of the plurality of raw materials; and
a step of inputting input data based on partial structure input data for each of the plurality of raw materials on which the blending ratio data is reflected to a machine learning model.

7. A property prediction program for predicting properties of a material based on a plurality of raw materials having a known partial structure, the program allowing a computer to execute:
a step of receiving at least input of partial structure data for specifying the known partial structure of each of the plurality of raw materials and blending ratio data indicating a ratio of blending of each of the plurality of raw materials;
a step of generating partial structure input data indicating the known partial structure, on the basis of the partial structure data for each of the plurality of raw materials;
a step of reflecting blending ratio data relevant to the plurality of raw materials on partial structure input data of the plurality of raw materials; and
a step of inputting input data based on partial structure input data for each of the plurality of raw materials on which the blending ratio data is reflected to a machine learning model.
